Europäisches Patentamt

European Patent Office

Office européen des brevets

(11) Publication number: 0 350 466
A2

# (12) EUROPEAN PATENT APPLICATION

(21) Application number: 89830309.4

(22) Date of filing: 04.07.89

(51) Int. Cl.⁵: **A 61 L 2/02**

(30) Priority: 06.07.88 IT 353088

(43) Date of publication of application:
10.01.90 Bulletin 90/02

(84) Designated Contracting States:
CH DE ES FR GB GR LI SE

(71) Applicant: CASTELLINI S.p.A.
Via Saliceto, 22
I-40013 Castelmaggiore (Bologna) (IT)

(72) Inventor: Castellini, Franco
Via Bellinzona, 60
I-40135 Bologna (IT)

Lenzi,, Plinio Maria
Via Leonello Spada, 47
I-40129 Bologna (IT)

(74) Representative: Lanzoni, Luciano
c/o BUGNION S.p.A. Via Farini, 37
I-40124 Bologna (IT)

(54) A method and apparatus for the cold sterilization of surgery instruments, in particular dental surgery instruments.

(57) The method and apparatus are designed to permit of sterilizing surgical instruments (1) by inducing electrolysis with a steady d.c. current of between 2.5 and 3.5 A in a water solution having pH-value between 5 and 7.5 and a concentration of chloride ions between 0.45 and 0.85g/l; the electrolyte (2) is buffered with each sterilizing cycle completed, and its condition monitored continuously.

FIG 2

## Description

### A method and apparatus for the cold sterilization of surgery instruments, in particular dental surgery instruments

The invention relates to a method of sterilizing surgical instruments under cold conditions, in particular dental surgery instruments, and to an apparatus for the implementation of such a method. The necessity for sterilizing surgical instruments is one of great importance, and most especially in environments where such instruments are brought directly or indirectly into contact with a major carrier of infection such as blood, saliva etc... Such is the case in environments where even small surgical operations are carried out, as for example, the dental surgery.

The sterilization of surgical instruments (that is, the entire range of items including handpieces, bistouries, tweezers, implements etc.) is essential, especially in situations where several patients are treated by a dentist within a short period of time, to the end of avoiding any transmission of infection from one patient to another.

The current state of the art in sterilization of surgical instruments offers three different methods. A first method makes use of gamma rays. This is a familiar technique already used in sterilizing disposable syringes, and notably effective from the standpoint of the results obtainable; nonetheless, one has the drawback that the apparatus required for its implementation is costly, and none too practical inasmuch as operation is necessarily subject to certain precautions.

In the second method, sterilization takes place in an autoclave; the instruments are generally put in a special bag that is fastened and placed inside the pressure vessel, submerged in a suitable liquid in certain instances. Sterilization is brought about in this case by the high temperature with which the instruments are invested.

Apparatus for this second method is less costly and more practical, though considerable disadvantages are encountered, connected with the high temperature to which the instruments are subjected, and the time needed to effect a sterilization. High temperature can in fact produce deformation in an instrument fashioned with parts in plastic, to the extent that its usefulness is affected, and in the case of a bistoury, may counteract the effects of hardening and cause the cutting edge to become dulled. The third method in question envisages immersion of the instruments in a solution of 2%-glutaraldehyde for a period of not less than three hours duration, in which case a substantial supply of instruments becomes necessary if work is to proceed reasonably uninterrupted.

Accordingly, the object of the invention is to set forth a method that will enable sterilization to be effected using low-cost, functional apparatus, and without any of the drawbacks mentioned above. The stated object is achieved with a method and a relative apparatus as characterized in the appended claims, through the adoption of which it is possible to sterilize instruments in cold conditions -viz, by generating active chlorine at a given concentration.

One advantage of the invention consists essentially in the fact that sterilization is accomplished by a simple and safe method, since electrolysis occurs at ultra-low concentration, at a controlled pH-value, and with a high relative production of chlorine. Another advantage given by the invention is that of the speed of the sterilizing operation. All that is required is to place the instruments in a receptacle and immerse the receptacle in the electrolyte; the cycle is then standard, effected automatically and with all the variable factors in play accounted for. A further advantage of the method disclosed is that it ensures faultless operation and a fully effective sterilization, without occasioning any damage to the instruments.

The invention will now be described in detail, by way of example, with the aid of the accompanying drawings, in which:

fig 1 is a plan of the apparatus according to the invention, showing the tank without its lid and without the receptacle in which surgical instruments are placed for sterilization;

fig 2 is the section through II-II in fig 1, showing the apparatus in its entirety and on smaller scale, ready to implement a sterilization cycle; fig 3 shows a block diagram of electrical components forming part of an apparatus as in figs 1 and 2.

The method of sterilizing instruments according to the invention comprises the steps of:

-immersing the soiled instruments 1 in a bath of electrolyte 2 consisting in a water solution having pH-value of between 5.0 and 7.5 and a concentration of chloride ions between 0.45 and 0.85g/l;

-inducing electrolysis for a period of set duration to the end of forming active chlorine;

-immediately removing and drying the instruments 1, having first rinsed at least those fashioned in materials other than plastic.

The electrolytic reactions that take place are the self-same classic reactions as those induced in the manufacture of chlorine, hydrogen and caustic soda by industrial methods -i.e. with chlorine produced at the anode, and hydrogen at the cathode, and the formation of alkaline hydrate in solution.

The aforementioned concentration of Cl- chloride ions in the electrolyte will be the lowest possible in order to avoid damage to the instruments 1 being sterilized, and sufficient to give place, in steady operation, to a concentration of active chlorine in the tank quantifiable at 100ppm $\pm$10%.

It is thus the active chlorine that constitutes the sterilizing agent; to ensure maximum effectiveness however, the chlorine needs to operate in an acid environment, or at all events, one having a pH-value lower than 7.5.

In such conditions, more exactly, chlorine is present as HOCl (considered to be the most active) whereas above pH 7.5, a separation occurs into OCl-, occasioning a drastic loss of sterilizing power. On the other hand, too low a pH-value can adversely affect the material in which the instruments happen to be fashioned, and accordingly, the electrolyte 2 is made to operate within the margins of pH 5 to 7.5 as aforementioned.

The electrolyte 2 is initially a water solution having a concentration of chlorine ions between 0.45 and 0.85g/l and a pH-value of between 5 and 7.5, and must be conditioned in readiness to effect a given number of sterilization cycles. This is accomplished by adding a first concentrated solution to the distilled or demineralized water used in preparation of the sterilizer, which contains chloride ions in a quantity calculated to give the right concentration in the tank; this first solution will be described in due course.

The pH-value mentioned is subject to change during operation, however, due to the continual formation of alkaline hydrate at the cathode, and if provision is not made to compensate such a contingency, the value will rise quickly to 8 and higher. To avoid such an occurence, a further solution, containing an acidifier, is admixed to the electrolyte with each sterilization cycle effected, thereby offsetting the rising alkaline content and maintaining the pH-value between 5 and 7.5. Such a step consists in the addition of a second and more dilute solution which, besides controlling pH, also carries a quantity of Cl- chloride ions that is calculated to compensate for the reduced proportion of chloride ions in the electrolyte 2 and hold the concentration of chlorine at a steady level. The second solution will also be described in due course.

To ensure best possible generation of the active chlorine, it is envisaged that electrolysis will be induced by supplying d.c. power at a steady currect (between 2.5 and 3.5 A per litre of electrolyte) and variable voltage, say between 4 and 8 V, according to conductivity and absorption of the electrolyte. This fixed current method has the advantage of ensuring a constant rate of electrolysis regardless of the temperature of the electrolyte bath, inasmuch as electrolytic activity is directly related to the number of Amperes generated, and in this instance, the number remains constant.

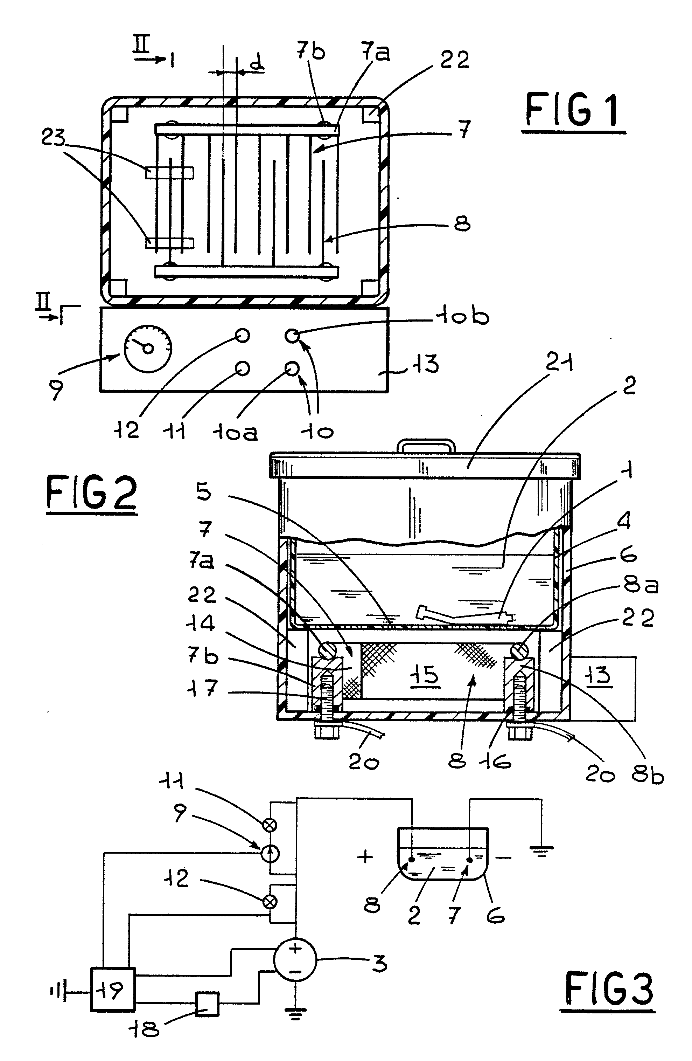

Referring now to fig 2, apparatus suitable for implementing the method according to the invention comprises:
-a receptacle 4, in which instruments 1 requiring sterilization are contained;
-a tank 6, with a removable lid, accommodating the receptacle 4 and filled with electrolyte 2;
-a pair of electrodes 7 and 8, incorporated into the base of the tank 6 beneath the receptacle 4 and connected to a generator 3 of electric current;
-a timer 18, capable of switching the generator 3 on and off;
-monitoring and control means 9, 10, 11, 12 and 19 capable of switching on the timer 18 and switching off the generator 3.

The receptacle 4 and tank 6 are both embodied in a material unaffected by the chlorine, such as ABS, PVC, polypropylene etc., and the receptacle 4 will be perforated at least at bottom. These perforations take the form of a set of holes 5 in the example of fig 2, though the bottom of the receptacle 4 might equally well be embodied in mesh, allowing the electrolyte a better circulation.

The tank 6 will be of some 15 litres capacity, and shaped to accommodate the receptacle 4 with its bottom raised a suitable height from the base. Comparing fig 1 and fig 2, it may be observed that the tank 6 appears as a parallelepiped with two electrodes, negative 7 and positive 8, centrally located at bottom. 13 denotes a base projecting from from one end of the tank 6, which can serve to accommodate the generator 3 and the monitoring and control means 9, 10, 11, 12 and 19 (see fig 1). 22 denotes a set of blocks located at the base of the tank 6; it is on these blocks that the bottom of the receptacle 4 is made to rest, thus allowing the electrolyte 2 to circulate freely beneath.

According to the invention, the tank 6 contains an electrolyte 2 consisting in a water solution of the following preferred composition:

| | |
|---|---|
| -NaCl | 0.75 ... 1.4g/l, equivalent to a concentration of Cl- chloride ions between 0.45 and 0.85g/l |
| -Citric acid | 0.00 ... 1.00g/l |
| -Sodium citrate | 0.00 ... 1.00g/l |
| -Demineralized $H_2O$ | by volume |
| -pH-value | 5.0 ... 7.5 |

Given that the chloride ion is the donor, it becomes possible to express the anion concentration only, provided that in replacing sodium, one of the alkali or alkaline earth metals is utilized.

In whichever instance, the basic values must remain:
Cl- chloride ions: 0.45 ... 0.85g/l
pH value 5.0 ... 7.5
Thus, the use of two distinct solutions is envisaged in implementing a method according to the invention: the one for initial conditioning of the electrolyte, the other for subsequent buffering and pH control. In a preferred formula, the first concentrated solution will be a water solution having a pH-value of between 3.5 and 4.5 and a concentration of Cl-chloride ions between 150 and 300g/l. The second, or buffer solution is likewise a water solution, but with a concentration of Cl- chloride ions of between 5 and 10g/l, and an acidifier the quantity of which will be sufficient to ensure that the pH-value of the electrolyte 2 remains within the limits stated. An advantageous acidifier would be citric acid, included in the buffer solution at a concentration of between 10 and 20g/l, such that each addition will increase the quantity in the tank by between 0.015 and 0.045g/l.

The negative electrode or cathode 7, is embodied in activated titanium, and the positive electrode, or anode 8, in titanium; both consist in a plurality of parallel screens denoted 14 and 15 respectively. The surface area of the screens 14 and 15 needs to be generous in order to guarantee a better and more effective electrolysis; preferred dimensions would be 0.8 ... 1.2dm$^2$ per litre of electrolyte for the cathode screens, and 0.4 ... 0.6dm$^2$ per litre for the anode.

The two sets of screens 14 and 15 are vertically disposed and set apart at an advantageous distance $\underline{d}$ (see fig 1) between the two electrodes 7 and 8; the distance d most suitable is approximately 3mm. In a preferred embodiment, each anode screen is located between two parallel cathode screens, and the 3mm spacing on either side is ensured by insulated distance pieces 23.

7a and 8a denote a pair of horizontally disposed round section bars to which the screens 14 and 15 are fastened along one edge, say, by welding; these bars will be fashioned in the same material as the screens themselves. Each bar 7a and 8a is bent downwards at one end, and terminates in a butt that affords a tapped hole encircled by a seat in which to lodge a seal 16.

17 denotes either one of two fixing screws freely insertable through holes in the base of the tank 6; each screw 17 engages in the hole of a relative bar 7a and 8a, and serves also to clamp the the end of a relative lead 20 connecting with the generator 3. Thus, the screws 17 perform a dual role, tightening the bars 7a and 8a against the base of the tank 6 and rendering the holes watertight by compressing the relative seals 16, and connecting the electrodes 7 and 8 to their power source, the generator 3. The generator 3 delivers d.c. power of which the current component remains steady (at between 2.5 and 3.5 A per litre of electrolyte) whilst the absolute value of the voltage is variable; monitoring and control means used in the apparatus comprise:

-a pair of buttons 10 serving to activate the apparatus;

-an instrument serving to measure the absorption level of the electrolyte 2 consisting, for example, in a voltmeter 9 capable of measuring voltage at the terminals of the electrodes 7 and 8;

-two acoustic and/or luminous indicators 11 and 12, the first wired in series with the voltmeter 9 and serving to signal excessive conductivity through the electrolyte 2, the second wired in parallel with the power circuit supplying the electrodes 7 and 8 and serving to signal insufficient absorption;

-a timer 18 serving to switch the generator 3 on and off;

-a controller, denoted 19, governing the entire range of operations, which may be programmable or otherwise.

A description now follows of the sterilization procedure, adopting the method according to the invention and utilizing an apparatus as described above for its implementation, assuming switch-on at the start of surgery hours.

The user first pours a prescribed quantity of the initial conditioner solution into the clean tank 6, covers the apparatus with the lid 21, and depresses the "condition" button 10a, whereupon the relative process will be set in motion. This preparatory step has a set duration of 12...15 min approx, which is clocked by the timer 18, and visual evidence of its progress is given by an indicator (not illustrated); on completion, another indicator lights up to signal "stand-by" status, and the apparatus is ready to sterilize. Termination of the conditioning process has the effect of enabling the controller 19 for subsequent sterilization, and the cycle can thus be activated at any given moment. Sterilization remains possible as long as the controller 19 is enabled, and the electrolyte will stay "conditioned" for up to four hours without being used.

The steps of the sterilization cycle occur as follows. Instruments 1 to be sterilized are washed in accordance with legal requirements and placed in the receptacle 4, whereupon the prescribed quantity of buffer solution is poured into the tank 6, and the receptacle 4 is lowered into the tank and set on the blocks 22.

Having made certain that the instruments are fully immersed in the electrolyte 2, the user can replace the lid 21 and depress the "sterilize" button 10b. Current now flows from the generator 3 for a set duration (5...7 min), controlled by the timer 18. A further indicator light confirms that sterilization is in process, and completion of the sterilization cycle is signalled by yet another indicator, as well as by an acoustic signal.

With sterilization completed, the receptacle 4 is removed without delay, and the instruments rinsed off and dried. Rinsing is indispensable for those instruments fashioned in material that might be susceptible to oxidation (even the stainless steel specifically formulated for surgical implements), though not imperative for plastic materials. At this juncture, the apparatus is ready for another sterilization cycle.

The indicator denoted 11 will light up in the event that the electrolyte 2 should become saturated with repeated cycles, or that an error is detected in the formulation of fresh electrolyte 2, i.e. registering too high a concentration, or containing water that has not been distilled or demineralized for whatever reason, accidental or otherwise.

Should the indicator 11 light up, the electrolyte 2 must be emptied out and replaced with fresh. The indicator denoted 12, on the other hand, will light up in the event that there is insufficient current absorbed. This can occur if the electrolyte is prepared with a quantity of conditioner less than that prescribed, or if the anode 8 suffers major damage; in either instance, the apparatus would not be able to perform dependably, though in the former instance it will suffice to add a quantity of the conditioner sufficient to restore normal operation. The voltage component of the d.c. output is variable between 4 and 8 volts, and accordingly, can serve as an indicator of the state of the electrolyte, capable of triggering a warning signal to denote minimum or maximum absorption in the event that the value should register respectively above or below a set threshold. In the event, for example, with current assumed as par, that voltage should drop below 5V, this would signify an excessive absorption, and the relative indicator 11 will be activated by the monitoring circuit; contrariwise, where voltage rises above the prescribed upper limit (signifying low conductivity of the electrolyte) the minimum absorption indicator 12 will light up. In selecting the upper voltage threshold, account must also be taken of the state of the electrodes, which will deteriorate progressively if subjected to excessive voltage levels; inefficient electrodes cause the system to lose conductivity further, regardless of other circumstances, with the result that voltage rises above the prescribed upper limit and triggers the minimum absorption indicator. To best advantage, the conditioner solution will be supplemented with a small quantity of surface active agents capable of improving penetration of the electrolyte into the cavities and gaps of the items sterilized, and generating a mild cleansing action. In an alternative embodiment of the apparatus, the controller 19 might comprise a CPU, in which case one button 10 only would suffice to set the cycle in motion.

## Claims

1) A method for the cold sterilization of surgical instruments, in particular dental surgery instruments characterized in that it comprises the steps of:
-immersing the instruments (1) totally in a bath of electrolyte (2) consisting in a water solution having pH-value of between 5.0 and 7.5 and a concentration of chloride ions between 0.45 and 0.85g/l;
-inducing electrolysis for a period of set duration utilizing a d.c. power output of which the current component remains constant at between 2.5 and 3.5 A per litre of electrolyte, and the voltage component is variable in absolute value to match conductivity and absorption of the electrolyte (2).

2) A method as in claim 1, wherein the step of totally immersing the instruments (1) is preceded by a step of buffering the electrolyte (2), consisting in the admixture of a solution containing an acidifier sufficient in quantity to maintain the pH-value within the prescribed limits.

3) A method as in claim 1, wherein the first sterilizing cycle is preceded by an initial step of electrolysis effected for a prescribed duration to the end of conditioning the electrolyte (2), which consists in a water solution with pH-value of between 5 and 7.5 and a concentration of chloride ions between 0.45 and 0.85g/l.

4) A method as in claim 1, wherein the step of inducing electrolysis continues for a duration of between 5 and 7 minutes.

5) A method as in claim 3, wherein the duration of the conditioning step is between 12 and 15 minutes.

6) A method as in claim 1, wherein the step of inducing electrolysis is followed by the steps of immediately removing the instruments (1) from the electrolyte (2) and drying them, having first rinsed at least such instruments (1) as may be fashioned in materials other than plastic.

7) A method as in claim 2 wherein the acidifying element is citric acid.

8) Apparatus for the cold sterilization of surgical instruments, in particular dental surgery instruments characterized in that it comprises:
-a receptacle (4), perforated at least at bottom, in which the instruments (1) requiring sterilization are contained;
-a tank (6), accommodating the receptacle (4), which has a removable lid and is of capacity such as to contain a predetermined quantity of electrolyte (2);
-a pair of electrodes (7, 8) situated at the base of the tank, beneath the receptacle, and connected to a generator (3) of electric current;
-a timer (18), capable of switching the generator (3) on and off;
-monitoring, control and indicator means (10, 11, 12, 19) capable of switching on the timer (18), and of switching off the generator (3) upon detection via a measuring instrument (9) of excessive or insufficient absorption of energy by the electrolyte (2).

9) Apparatus as in claim 8, comprising electrodes (7, 8) that consist in a plurality of parallel screens (14, 15) rigidly attached to respective bars (7a, 8a) fastened to the tank, wherein the negative electrode (7) exhibits a surface area of 0.8 ... 1.2 $dm^2$ per litre of electrolyte, and the positive electrode (8) exhibits a surface area of 0.4 ... 0.6$dm^2$ per litre of electrolyte (2).

10) Apparatus as in claim 9, wherein the electrodes (7, 8) are disposed parallel one to the other with the respective cathode and anode screens (14, 15) set in alternation, each anode screen lying between two cathode screens, and spaced apart approximately 3mm one from the next by distance pieces (23) fashioned in an insulating material.

EP 0 350 466 A2

FIG 1

FIG 2

FIG 3